(19) Europäisches Patentamt
European Patent Office
Office européen des brevets

(11) **EP 1 601 623 B1**

(12) **EUROPEAN PATENT SPECIFICATION**

(45) Date of publication and mention of the grant of the patent:
**11.04.2007 Bulletin 2007/15**

(21) Application number: **04712976.2**

(22) Date of filing: **20.02.2004**

(51) Int Cl.:
**C03C 10/00** (2006.01)          **A61F 2/28** (2006.01)
**C03C 3/00** (2006.01)

(86) International application number:
**PCT/BR2004/000015**

(87) International publication number:
**WO 2004/074199 (02.09.2004 Gazette 2004/36)**

(54) **PROCESS AND COMPOSITIONS FOR PREPARING PARTICULATE, BIOACTIVE OR RESORBABLE BIOSILICATES FOR USE IN THE TREATMENT OF ORAL AILMENTS**

VERFAHREN UND ZUSAMMENSETZUNGEN ZUR HERSTELLUNG VON TEILCHENFÖRMIGEN, BIOAKTIVEN ODER RESORBIERBAREN BIOSILIKATEN ZUR VERWENDUNG BEI DER BEHANDLUNG VON MUNDBESCHWERDEN

PROCEDE ET COMPOSITIONS PERMETTANT DE PREPARER DES BIOSILICATES PARTICULAIRES, BIOACTIFS OU RESORBABLES UTILISES DANS LE TRAITEMENT D'AFFECTIONS ORALES

(84) Designated Contracting States:
**AT BE BG CH CY CZ DE DK EE ES FI FR GB GR HU IE IT LI LU MC NL PT RO SE SI SK TR**

(30) Priority: **20.02.2003 BR 0300644**

(43) Date of publication of application:
**07.12.2005 Bulletin 2005/49**

(73) Proprietors:
• **Universidade Federal de Sao Carlos**
**13565-905 Sao Carlos - SP (BR)**
• **Universidade De Sao Paulo-USP**
**05508-900 Sao Paulo - SP (BR)**

(72) Inventors:
• **DUTRA ZANOTTO, Edgar**
**13561-160- Sao Carlos- SP (BR)**

• **RAVAGNANI, Christian**
**14700-000- Bebedouro- Sao Paulo (BR)**
• **PEITL FILHO, Oscar**
**13560-320- Sao Carlos- SP (BR)**
• **PANZERI, Heitor**
**14051-160- Ribeirao Preto- SP (BR)**
• **GUIMARAES LARA, Elza Helena**
**14051-230- Ribeirao Preto- SP (BR)**

(74) Representative: **Franzolin, Luigi et al**
**Studio Torta**
**Via Viotti 9**
**I-10121 Torino (IT)**

(56) References cited:
**US-A- 5 981 412**          **US-B1- 6 244 871**
**US-B1- 6 338 751**          **US-B1- 6 342 207**

EP 1 601 623 B1

## Description

The present invention relates to a process for preparing particulate, bioactive or resorbable crystalline silicates (biosilicates), as well as to the compositions used to prepare said particulate bioactive or resorbable silicates, to the biosilicates *per se* and to the use of such products in the treatment of oral ailments. More specifically, the present invention relates to processes for preparing particulate, bioactive or resorbable silicates having particle size distribution and crystalline phases defined on the basis of certain compositions that are homogenized and melted, the molten liquid being then cooled and solidified. The so-obtained vitreous pieces are crystallized by means of an isothermal or once-through treatment; with the crystallized pieces being milled to the desired particle size distribution. The temperature and residence time of the silicate in said treatment should be strictly determined so as to obtain a controlled and practically complete crystallization (that is having residual vitreous phase lower than 0.5%) of the biosilicate stock. Alternatively, the compositions are homogenized and melted, the molten liquid being then cooled and solidified, milled to the desired particle size distribution and the obtained powder submitted to isothermal treatment. Still alternatively, the compositions can be submitted to a two-step thermal treatment, the powder being obtained before or after the thermal treatment. The biosilicate products of the invention having larger particle sizes are endowed with bioactivity, that is, quick formation of an hydroxycarbonate apatite (HCA) layer on the biosilicate surface in the presence of body fluids, that layer promoting bonds with bone or dental tissues. Biosilicate surface reactions in the presence of saliva release ions that aid in dental tissue remineralization processes. The inventive biosilicate products having lower particle size or crystalline phases having higher biological activity are also resorbable, that is, they are dissolved by the body fluids or gradually replaced by tissue when in contact with it, with the degradation products being non-toxic, easily metabolized by the tissue and not harmful at all.

The silica-based compositions of the invention additionally contain calcium, phosphorus and sodium ions, and optionally potassium, fluorine, lithium, tin, strontium, iron, magnesium, boron, aluminium and zinc. The resorbable biosilicate products of the invention show high stability in some dentifrice solutions, making them able to be used in the treatment of several oral ailments using oral hygiene and treatment products as vehicles.

## BACKGROUND INFORMATION

Basically, treatments for dentine desensitizing make use of the principle of desensitization of pulp mechano-receptors, of the occlusion of the dentine tubules or of both simultaneously, as taught in the articles by Bolden, T. E. "A desensitizing dentifrice with multiple oral health benefits formulated for daily use" *in* The Journal of Clinical Dentistry, v. 5, p. 68-70, 1994. Special Issue and Miller, S. et al. "Evaluation of a new dentifrice for the treatment of sensitive teeth" *in* The Journal of Clinical Dentistry, v. 5, p. 71-79, 1994. Special Issue.

According to Brazilian Applications P10007642, P19909831, P1980A777 and PI9503544, some tooth pastes employ a mixture of two components that are kept apart until the moment they are dispersed in the teeth. The first component is a potassium salt that, when present in the oral environment, promotes the release of $K^+$ ions. The potassium ions have the ability to diffuse towards the interior of the dentine tubules and increase their concentration near the pulp nerves under the sensitive dentine. The continuous presence of $K^+$ causes an analgesic effect on the mechano-receptors close to the pulp nerves, being responsible for a desensitizing effect.

The second working element is a stannous salt in which $Sn^{2+}$ ions behave according to a mechanism similar to that of $K^+$, working in the sense to bring relief to the dentine sensitivity.

Besides the inconvenience of the need of two topic applications of these components, that should be kept apart until the dispersion moment, there is the need for the patient to keep a continued treatment in order to maintain a low level of dentine sensitivity. This is caused by the fact that the lower concentration of $K^+$ or $Sn^{2+}$ near the mechano-receptors will tend to reduce the analgesic effect provided for by such treatment. Therefore, this approach does not provide a long term solution for the problem of dentine sensitivity, and treatment discontinuity leads to return to pre-treatment sensitivity levels.

As reported in the articles by Bolden and Miller cited before, the desensitizing agent of some tooth pastes is a potassium salt combined to inorganic microparticles and a polymeric adhesive. Inorganic particles, such as, for example, silica, having the same diameter of the dentine tubules may be mechanically attached to the opening of said tubules. The polymeric adhesives present in the dentifrice are able to adhere to the dentine surface as well as to the inorganic particles, imprisoning said particles and reinforcing their adherence to the dentine tubules and to the teeth surface, thus forming a protective layer. However, the adhesive force of these particles as well as of the polymeric adhesive to the dental surface is relatively weak and they are easily detached by brushing as well as by the cycles of the oral pH. Thus, this is also not a long lasting treatment and needs constant application by the patient. The treatment interruption causes the return of the dentine sensitivity to the normal levels.

International Publication WO93/25183 teaches that the promotion of the dentine sensitivity reduction may benefit from the use of silica, alumina, synthetic resins and calcium insoluble salts microparticles that are incorporated into an oral antiseptic.

During use by the patient, such microparticles would be able to mechanically attach to the dentine surface so as to hinder the movement of fluid in the dentine interior. Again, the adhesion of such particles to the tubule openings is mechanical only, and thus they can be easily dispersed by oral hygiene practice or during food intake.

According to Brazilian applications P19609829, P19711416, P19711339 and P19610258, and US patent 6,436,370, methods for the treatment of lesions caused by cavities, crevices calcification and dentine sensitivity treatment are also suggested based on soluble or slightly soluble calcium- and phosphate -derived salts, that may be mixed to acidic or basic solutions and that are based on the principle of releasing calcium and phosphorus ions to promote tooth remineralization and the occlusion of the dentine tubules.

Such methods provide a rise in the temporary concentration of calcium and phosphorus ions in the oral environment, proportional to the application period, which is generally too short to allow that the remineralization reactions occur in a satisfactory way, due to the slow rate of absorption of such, ions by the tooth hydroxyapatite crystals..

A further approach to the treatment of oral ailments is related to bioactive glasses.

Bioactive glasses are vitreous materials having biological activity, that is, in the presence of an aqueous medium, ions are leached from the surface of such materials, then a nanoporous silica-gel layer is formed, serving as a support, and finally hydroxyapatite (HA) is formed within such nanopores and on the surface, by diffusion of calcium and phosphorus ions from the glass or from the aqueous medium to the surface. This way, bioactive glasses are able to fix themselves to the surface of bone tissues and to the tooth, so as to establish a strong and long lasting chemical bond.

When a bioactive glass contacts the body fluids (for example, blood plasma) there is at first leaching of $Na^+$ cations and replacement of same in the glass structure by $H^+$ or $H_3O^+$ cations (stage I). This causes a rise in the local pH causing the breaking of Si-O-Si and silicon is released into the solution as $Si(OH)_4$ (stage II). If the local pH is lower than 9.5, $Si(OH)_4$ condenses and silica $(SiO_2)$ re-polymerizes on the glass surface, forming a silica-gel layer (stage III).

The silica-gel open structure allows that the ion exchange between the glass and the solution continues to occur. Calcium and phosphorus ions diffuse from the glass through the silica-gel layer, those added to the calcium ions and to the phosphates present in solution forming on the glass surface a layer of amorphous calcium phosphate (stage IV).

According to the glass bioactivity level, such reactions initiate a few minutes after the glass contacts the fluid. After the thickness increase of the amorphous silica-gel and phosphate layers, this latter incorporates hydroxyl, carbonate or fluoride ions, starting the crystallization into apatite (stage V).

However, in order to a material be considered bioactive the surface reactions should occur at a controlled rate. For example, if the leaching process of the $Na^+$ ions (stage I) is too slow there is not a sufficient rate of glass structure disruption so as to produce the silica-gel layer in the required amount to form the HCA layer on the surface.

On the other hand, if these reactions occur very quickly so that the material is quickly dissolved, there is the continuous replacement of the material by the bone tissue and the material is considered as resorbable.

In view of the bioactive properties of these glasses, it was suggested to use them to treat the problem of dentine sensitivity.

Thus, the treatment for the dentine sensitivity taught by Brazilian Application PI9707219 as well as by US patents 6,338,751, US 6,244,871 and US 5,735,942 suggest the use of the bioactive glass in particulate form, where the particle size distribution of a large fraction of the powder is between 1 and 2 microns, this indicating the effective ability of the glass to penetrate in the small channels or to deposit on the dentine surface, so that in the presence of the oral fluids hydroxycarbonate apatite formation may start on the dentine surface and chemically bond itself to the dental tissue.

The above-cited patents teach the use of bioactive glass particles having a particle size distribution < 90 microns. Thus, lower-than-2 micron particles can penetrate the dentine tubules and promote the occlusion of such tubules while the larger particles can adhere to the tooth surface and work as calcium and phosphorus ions suppliers for the hydroxycarbonate apatite formation reactions on the surface of the smaller particles positioned within the dentine tubules.

The above-cited patents also teach the incorporation of such particulate bioactive glasses to tooth pastes and gel, these being the vehicles for particle distribution to the dentine surface during brushing.

A drawback to the use of the bioactive glass under these conditions is the high cutting power of the particles caused by their extremely irregular, pointed and sharp shape, formed when the particles are fractured during the powder production process. These milled glass particles can cause irritating micro-cuts in the gengiva, with the sensation of augmented sensitivity.

US patent 5,891,233 teaches the clinical use of the particulate bioactive glass for treating dentine sensitivity as well as exposed roots by mixing, just before use, the glass to a physiological fluid, and applying the mixture locally to the tubules of the exposed dentine regions. Then, the treated regions are covered with a protecting mold to avoid powder dispersion.

US patents 6,342,207 and US 6,190,643 teach the use of bioactive particulate glass of particle size < 100 microns for the prevention and treatment of oral ailments caused by pathogenic microorganisms, such as for example cavities and gingivitis.

The dissolution of the particles of such bioactive glass causes a great rise in the osmotic pressure and pH - caused by the release of sodium and calcium ions in solution. These two effects, combined to the high concentration of $Ca^{2+}$ ions in the liquid make that pathogenic microorganisms are eliminated from the oral environment, eliminating cavity-causing bacteria, gingivitis, plaque and other pathologies.

However, according to studies reported in US patents 6,342,207 and US 6,190,643 it was found that non-pathogenic *Streptococci sanguis* bacteria could not be eliminated. This is due to the fact that those bacteria show better resistance to high osmotic pressures than pathogenic bacteria. The bioactive glass described in these US patents should be mixed to water or to an aqueous solution and applied to the teeth and areas to be treated. Thus, the suggested use is restricted to clinical applications.

The behavior of non resorbable, bioactive monolith glass-ceramics used in bone implants is similar to that of bioactive glasses. As in bioactive glasses, in bioactive glass-ceramics an HCA surface layer is formed, being responsible for the establishment of a strong bond between these materials and the bones of a patient a few hours after their surgical implantation. These glass-ceramic materials contain silica and calcium oxides, sodium and phosphorus and are useful for bone prosthesis. US patent 5,981,412 describes such a bioactive material, having crystallinity between 34 and 60 percent by volume and a 1 $Na_2O.2CaO.3SiO_2$ crystalline phase.

According to US patent 5,981,412, the bioactivity level in bioactive glass-ceramics depends on the chemical composition, percent of crystallinity, crystalline phase(s) and microstructure. The mechanical properties depend on the crystallized fraction, crystal size, crystalline phase(s) and crystal shape.

In US patent 5,981,412 the crystallization of a glass into a glass-ceramic is effected in two stages, that is, nucleation and crystal growth. A glass nucleation thermal treatment at varied temperature and times yields a sample having a matrix with dispersed crystalline nuclei.

In a second step of the thermal treatment the glasses containing the crystalline nuclei generated in the first step are submitted to a higher temperature, for a certain time, during which is promoted the growth of those nuclei. Optical microscopy assessments lead to the determination of the evolution of the crystal size for a specific temperature as a function of thermal treatment periods and the growth rate can be calculated.

Once the nucleation rate and crystal growth curves are obtained, a glass-ceramic having a crystallized volumetric fraction suitable for applying the material as solid pieces (monoliths) can be obtained, since partial glass crystallization provides a rise of their mechanical properties as compared to its vitreous form, while the bioactivity is kept as such. These features are relevant to the use of a monolithic bioactive material as a prosthesis.

Despite the fact that the glass-ceramic material taught in US patent 5,981,412 presents HCA formation mechanisms to promote the bond of the implant to the patient bone tissue, the material described therein is used as an implant in the shape of monolith pieces. Therefore, the teachings of said US patent do not describe nor suggest the bioactive, resorbable, crystallized powders of the present invention, that may be included in gels and mouth washings for the treatment of oral ailments. Besides, said US patent also does not contemplate the milling of the obtained glass-ceramics. Besides, the described monolithic glass-ceramics are strictly bioactive and are not resorbable.

The inventive biosilicates are preferably obtained by a practically complete crystallization of a vitreous material through thermal treatments in a single stage (isothermal) or, alternatively, in two stages, and further milling for obtaining a particulate material having a particle size distribution in the range of 30 to 0.1 micron, this procedure being different from the glass-ceramics object of US patent 5,981,412.

Alternatively, the biosilicates can be obtained by milling the vitreous material until the particle size distribution range between 30 and 0.1 micron is obtained followed by practically complete crystallization of the particulate material through a thermal treatment in one single stage, or alternatively in two stages, where the particle surface crystallization prevails.

Isothermal or alternatively two-stage treatments applied to the particulate biosilicates of the present invention make possible to obtain a crystallized material having one single or different crystalline phases (not only the $1Na_2O.2CaO.3SiO_2$ phase of the US patent 5,931,412) with different HCA formation rates, especially suitable for the treatment of the oral ailments described in the present specification, the described biosilicates being only bioactive or bioactive and resorbable, depending on the crystalline phase and on the particle size.

The inventive biosilicate, in particulate form, is incorporated into oral hygiene products, such as, for example, gels, dental creams, oral antiseptic liquids, saline solutions, artificial saliva, etc, for utilization in the practice of the everyday oral hygiene or in the dental clinic by a dentist. This way, the biosilicates are designed for external use in a continuous way and do not involve the need of surgery.

Therefore, in spite of the technical progress in this field, there is a need to develop a process for preparing particulate, bioactive or resorbable silicates having particle size distribution between 0.1 and 30 microns based on certain compositions that are homogenized and melted, the molten liquid being then cooled and solidified, followed by thermal treatment in one stage (isothermal), or in two stages, the temperature and residence time of the silicate during these treatments being such as to lead to a controlled crystallization and well-defined crystalline phases product, the obtained product being milled in the desired particle size distribution to obtain the biosilicate, or alternatively submitting the milled powder to isothermal treatment or in two stages, such process being described and claimed in the present application.

## SUMMARY OF THE INVENTION

Broadly, the process for preparing the particulate, bioactive or resorbable silicates of the invention from vitreous plates or frits comprises the following steps:

Submitting the vitreous plates or frits to a one-stage thermal treatment at such a temperature so as to simultaneously promote crystal nucleation and growth, a crystallized material being obtained;
Milling said crystallized material until the desired particle size distribution, obtaining the particulate, bioactive or resorbable, crystalline silicate.

Alternatively, the process of the invention comprises the following steps:

Milling the vitreous plates or frits in order to obtain a vitreous powder of desired particle size distribution; and
Submitting said powder to a one-stage thermal treatment at such a temperature as to simultaneously promote the crystal nucleation and growth, yielding a crystallized material.

Still alternatively, the thermal treatment can be carried out in two steps, one nucleation step and one crystal growth step, yielding ultimately the crystalline biosilicate.

In order to obtain the desired particle size distribution of the bioactive silicate, the material as plates or frits, either submitted to the two-stage thermal treatment or not, is reduced to a particle size preferably lower than 150 microns and the obtained grains are then wet milled, in an anhydrous organic liquid medium, such as isopropyl alcohol or acetone (not limited to those), making use of any suitable mill such as a planetary mill equipped with jar and agate balls, during 100 to 200 minutes, according to the specific chemical composition. According to the invention the biosilicate preferred particle size is between 0.1 and 30 microns.

The glass preparation is part of the state-of-the-art technique, and includes:

Weighing and mixing for about 20 minutes the different composition constituents;
Melting the mixed compositions at temperatures between 1200°C and 1500°C for 2 to 6 hours, obtaining an homogeneous liquid;

pouring the so-obtained homogeneous liquid:

c1) between two metallic bars and then pressing it to form the vitreous plates; or
c2) into distilled water for obtaining vitreous frits.

The mixing of the constituents is carried out according to usual, state-of-the-art techniques and devices that are well-known by the experts.

Useful compositions for preparing the inventive biosilicates comprise, in weight percentage, between 4.0 and 60% $SiO_2$, between 0 and 30 % $Na_2O$, between 0 and 30% $K_2O$, between 15 and 30% CaO, between 0 and 15% $CaF_2$, between 0 and 15% NaF, between 0 and 10% $Li_2O$, between 0 and 10% SnO, between 0 and 10% SrO, between 0 and 8% $P_2O_5$, between 0 and 6% $Fe_2O_3$, between 0 and 3% MgO, between 0 and 3% $B_2O_3$, between 0 and 3% $Al_2O_3$, and between 0 and 3% ZnO.

The use of the inventive biosilicates is directed to the treatment of oral ailments such as cracks, demineralization, and filling of teeth pores, and health recovery in *gingivitis.*

Therefore, the present invention provides a process for preparing particulate bioactive or resorbable silicates from vitreous plates or frits by one-step thermal treatment followed by milling until the desired particle size distribution, so as to obtain crystalline particles practically free of vitreous phase (that is, where the residual vitreous phase is lower than 0.5%) that could produce a cutting edge, this meaning minimal aggression to oral mucosae.

The invention provides further a process for preparing particulate bioactive or resorbable silicates from vitreous plates or frits by milling followed by a one-step thermal treatment so as to obtain crystalline particles practically free of vitreous phase that could result in a sharp, cutting edge, so as to avoid aggression to oral mucosae and excessive tooth wear.

The invention provides still a process for preparing particulate bioactive or resorbable silicates from vitreous plates or frits where the thermal treatment preceding or succeeding the glass milling is effected in two steps, a nucleation step and a crystal growth step, obtaining crystalline particles practically free from vitreous phase that could result in a cutting surface so as to avoid aggression to the oral mucosae and excessive tooth wear.

The invention provides further compositions based on silica and inorganic oxides on which are based the silicates that by milling and thermal treatment lead to the present particulate bioactive or resorbable silicates.

The invention provides further particulate bioactive or resorbable crystalline silicates that on being incorporated into gels and other products of oral hygiene are useful for the treatment of oral ailments, gingivitis and teeth pores, also reducing the dentine sensitivity.

## BRIEF DESCRIPTION OF THE DRAWINGS

FIGURE 1 attached illustrates infrared spectra of one of the biosilicate compositions of the invention before and after bioactivity tests in SBF K-9 liquid.
FIGURE 2 attached illustrates infrared spectra of a particulate biosilicate with a particle size distribution between 2 and 0.1 micron exposed at different periods in a gel used as an oral hygiene product.

FIGURE 3 attached illustrates micrographs of the tooth cervical area surface, the tooth having been treated with a biosilicate of particle size distribution between 20 and 0.1 micron. FIGURE 3A illustrates a 2,000 fold increase. FIGURE 3B illustrates a 10,000 fold increase. FIGURE 3C illustrates a 10,000 fold increase. FIGURE 3D illustrates a 3,000 fold increase. FIGURE 3E illustrates a 5,000 fold increase.

FIGURE 4 attached is a graph illustrating the pH rise of an aqueous solution with exposure period to an inventive biosilicate and to a glass of the same chemical composition and particle size distribution as the biosilicate. The particle size distribution of both is between 20 and 0.1 micron.

FIGURE 5 attached illustrates DSC thermograms, one thermogram for a composition free of $K_2O$ and a thermogram for a composition where all the $Na_2O$ is replaced by $K_2O$. This Figure illustrates the temperature intervals where are effected the thermal treatments for each composition.

## DETAILED DESCRIPTION OF THE PREFERRED MODES

### Process for preparing the particulate crystalline biosilicates

A first aspect of the invention is a process for preparing particulate crystalline biosilicates.

According to the invention, one mode of the process for preparing the particulate, crystalline biosilicates from vitreous plates or frits comprises the following steps:

Submitting the vitreous pieces to thermal treatment with the simultaneous promotion of the crystal nucleation and growth at temperatures between 400°C and 1050°C for 1 hour to 150 hours preferably between 500°C and 700°C for 3 hours to 100 hours, more preferably between 560°C and 670°C for 10 hours to 50 hours, obtaining a crystallized silicate;
Milling the crystallized silicate until the desired particle size distribution, and obtaining the particulate crystalline biosilicate.

The rather broad temperature and time ranges set forth in item a) are due to the huge variation in chemical composition that is possible according to the concept of the invention, with small variations in the chemical composition causing significant variations in the glass viscosity and therefore in the thermal treatment to be used to promote the crystallization.

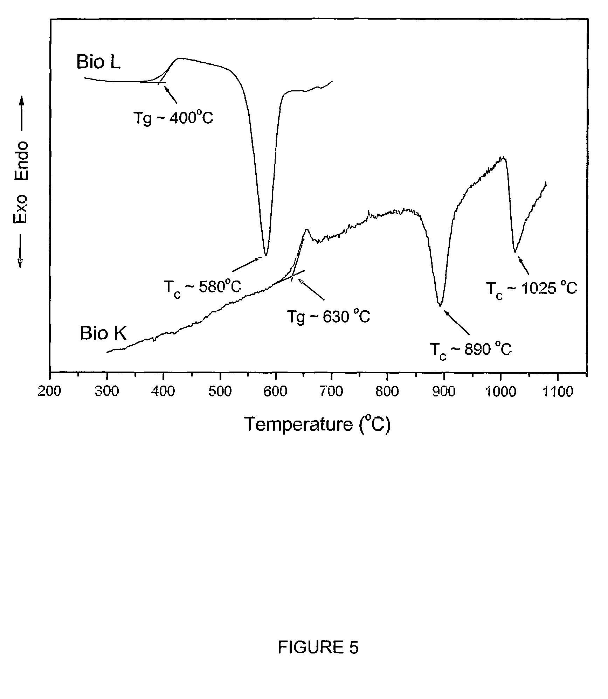

DSC (Differential Scanning Calorimetry) curves of Figure 5 illustrate the huge difference in thermal treatment temperature experienced for the crystallization of glasses of different compositions.

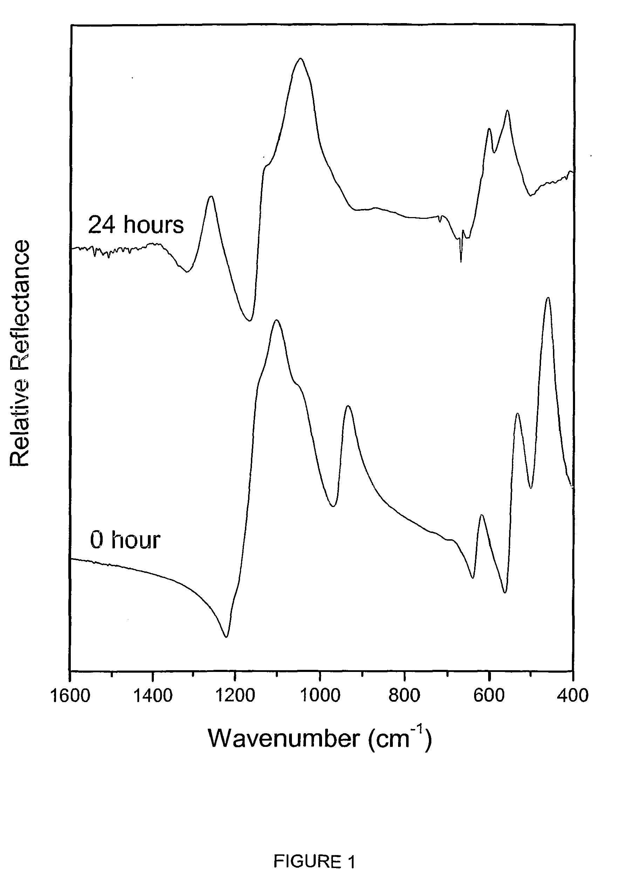

The thermal treatment for obtaining biosilicates for all the modes of the invention should be carried out between the glass transition temperature ($T_g$) and the crystallization temperature ($T_c$) shown in Figure 1. Thus, by varying the composition, these ranges can be extensively varied.

Figure 5 shows DSC curves for the following compositions in weight %:

| | Biosilicate L1: |
|---|---|
| $SiO_2$ | 44.9% |
| $Na_2O$ | 22.0% |
| CaO | 22.0% |
| $Li_2O$ | 7.4% |
| $P_2O_5$ | 3.7% |
| | Biosilicate K1: |
| $SiO_2$ | 48.5 |
| $K_2O$ | 23.75 |
| CaO | 23.75 |
| $P_2O$ | 4.0 |

For the potassium-containing compositions the thermal treatment temperature range is even broader, since the crystallization of such glasses occurs at higher temperatures.

Besides, the thermal treatment is largely varied for obtaining different crystalline phases as well as the final biosilicate microstructure. For example, in Figure 5 the DSC thermogram for the Biosilicate K1 composition shows the crystallization of two crystalline phases at the crystallization temperatures, $T_c$, of 890°C and 1025°C.

Alternatively, the inventive process comprises:

Milling the vitreous plates or frits to obtain a vitreous powder having the desired particle size distribution; and
Submitting the so-obtained powder to a thermal treatment, simultaneously promoting the crystal nucleation and growth at temperatures between 400°C and 950°C for 1 hour and 150 hours, preferably between 500°C and 700°C for 3 hours to 50 hours, more preferably between 540°C and 650°C, for 5 hours and 20 hours, so as to obtain crystallized particulate bioactive or resorbable silicates.

The duration of the thermal treatment periods is more or less long depending on the desired preferred crystalline phase and stem glass composition.

In the isothermal thermal treatment for obtaining a biosilicate, the crystal nucleation and growth is simultaneously obtained by submitting the vitreous material to temperatures above the glass transition temperature, $T_g$, that in the case of the present compositions varies between 400°C to 680°C. By varying the temperature and the time of the thermal treatment it is possible to vary the number and size of the crystals that are distributed through a certain biosilicate volume.

In order to obtain the biosilicate desired particle size distribution, the material as plates or frits, either previously submitted to the thermal treatment or not, is reduced to a particle size that is preferably smaller than 150 micron, but not being limited to that, and the obtained grains are then wet milled, in an anhydrous organic liquid medium, for 100 to 200 minutes, depending on the chemical composition and specific microstructure.

Anhydrous organic liquid media useful for the milling, but not limited to such, are isopropyl alcohol or acetone.

Mills that may be used for milling the biosilicate material, but not limited to such, are those of the planetary type having a jar and agate balls.

Alternatively, the process for preparing the particulate bioactive silicates of the invention from vitreous plates or frits comprises the following steps:

submitting the vitreous pieces to the two-stage thermal treatment, with the first stage of crystal nucleation at temperatures between 400°C and 850°C for 10 hours to 150 hours, preferably between 450°C and 680°C for 15 hours to 130 hours, more preferably between 500°C and 600°C, for 20 hours to 120 hours, and the second stage of crystal growth at temperatures between 500°C and 1050°C for 1 hour to 20 hours, preferably between 530°C and 700°C for 1 hour to 15 hours, more preferably between 560°C and 680°C for 1 hour to 10 hours, obtaining bioactive silicates having a controlled crystal size and practically free of vitreous phase;
milling the crystallized product until the desired particle size distribution, obtaining the bioactive crystalline particulate silicate.

Still a further alternative for the present process comprises:

a) milling the vitreous plates or frits in order to obtain a vitreous powder having the desired particle size distribution; and
b) submitting the so-obtained powder to a two-stage thermal treatment with the first crystal nucleation stage at temperatures between 400°C and 800°C for 1 hour and 50 hours, preferably between 450°C and 600°C for 1 hour to 30 hours, more preferably between 500°C and 580°C for 1 hour to 15 hours and the second stage, for crystal growth, at temperatures between 500°C and 900°C for 1 hour to 20 hours, preferably between 500°C and 680°C for 2 hours to 15 hours, more preferably between 560°C and 660°C, for 3 hours to 10 hours, obtaining particulate bioactive silicates having controlled crystal size.

The constituents of the silica-based compositions used in the process for preparing the present biosilicates should be at first weighed with the aid of precision scales and admixed in a planetary mixer for 20 minutes.

Then the constituents are melt in platinum crucibles, in an oven at temperatures varying between 1200°C and 1500°C, depending on the specific composition, for a period between 2 to 6 hours, in order to secure perfect homogenization.

At the end of this period, the molten liquid is poured in and pressed between two metallic bars for obtaining vitreous plates.

Alternatively, the molten liquid is poured into distilled water for obtaining vitreous frits.

The biosilicate crystallization eliminates an important drawback of the state-of-the-art vitreous milled powders, that is, the extremely irregular and sharp conchoidal fracture. That is why the powder in the vitreous form cuts the gengiva.

On the other hand the glass controlled crystallization according to the present process, employing any of the possible alternatives of thermal treatment set forth above in the present specification, eliminates the serious drawback of the vitreous powder having sharp and cutting edges.

According to the modes of the process for preparing bioactive silicate, for the crystallization of the already particulate glass, during the thermal treatment the glass atoms have a certain mobility that is a function of the viscosity, and therefore of the temperature at which the glass is kept. Aiming at reducing the surface energy, the particles tend to adopt a morphology that could satisfy a condition of lower surface area, tending to a sphere. Thus during the crystallization thermal treatment, the particle sharper contours are progressively rendered more rounded. In this way, it is possible to obtain particles of less acute and cutting ends.

An important aspect is the control of the crystallization time and temperature, since thermal treatments at higher temperatures or during higher times can also cause the particle sintering with the consequent particle size increase, this being undesirable. In this case, a further milling procedure is predicted, in order to effect the particle size adjustment.

Thus, for each composition the thermal treatment conditions are adjusted to produce the product ideal morphological features, while simultaneously preventing particle sintering.

Therefore, by manipulating the biosilicate composition, the time and temperature of the isothermal or two-step treatment of the silicate, it is possible to control the crystal size distribution, as well as to vary the amount of the obtained crystallized phases, resulting in a crystalline material.

Some of the crystalline phases obtained by the isothermal or two-phase treatments herein described are: $1Na_2O.2CaO.3SiO_2$, $2Na_2O.1CaO.3SiO_2$, $Na_2OCa$

$(PO_4)F$, $Ca_5Si_6O_{16}(OH)_2.8H_2O$. For compositions containing $SiO_2$, $Na_2O$, CaO and $P_2O_5$ with thermal treatments at temperatures higher than 800°C and for periods longer than 10 hours it is possible to obtain the apatite crystalline phase.

The various modes of the process for preparing biosilicates lead to biosilicate products of controlled crystal size. The fractures of such products tend to propagate through the crystal contours or by their cleavage plans. In low-energy milling, such as for example in ball mills, the fractures preferably follow the crystal contours while in high-energy milling these fractures follow the crystal contours as well as the crystal cleavage plans.

For the products resulting from all the predicted modes, in one or two-stage thermal treatment, the fractures of the crystalline particulate biosilicates of the invention differ from the fractures occurring in the surface of state-of-the-art bioactive glasses. The feature of the surfaces and plans of the biosilicate products of the invention is that they are more even or smoother, giving rise to edges that in spite of their angles are not sharp nor cutting, this rendering the present biosilicates especially adapted for use in the treatment of oral ailments through the practice of routine oral hygiene, by incorporating them into oral hygiene products.

According to the invention, the bioactive silicate particle size is between 0.1 and 30 microns. Optionally the particle size can be lower than 0.1 micron.

The process for preparing the particulate crystalline biosilicates of the invention leads to different crystalline phases, combined or isolated in such products, according to the temperature and time of the isothermal or two-stage treatment to which this material has been submitted. Such phases show different levels of biological activity. Through different isothermal or two-phase treatments it is possible to vary the relative fraction of each phase in the particulate biosilicate. The different compositions and crystalline phases suggested in the invention can be used separately as well as combined.

According to the invention, crystalline phases of higher biological activity show higher reaction rates with dental tissues and in the presence of body fluids, with a quick leaching of ions from the material surface and ion release into solution. Thus, the biosilicates made up of these phases are more quickly bound to the tissues and are progressively replaced by the surrounding tissue, causing the regeneration of such tissues.

Using them in a combined way, the crystalline phases of higher biological activity have a more direct action on the occlusion and coverage of the dentine tubules, filling of microfissures and lesions, as well as in the repairing of the dental enamel by progressive resorption and substitution of biosilicate by tissue.

And according to the invention, the crystalline phases having a relatively lower biological activity, but still of high bioactivity due to the slower surface reaction rates than the resorbable phases, are bound to the dental surface through the formation of a HCA layer.

Thus the higher sized particles and those of the bioactive crystalline phases, although not resorbable, keep longer in the oral environment, and can work mainly as ion suppliers for the remineralization and recalcification reactions for more prolonged periods, aiding in the dentine and tooth surface regeneration processes.

It is possible to vary the fractions of the different desired crystalline phases by varying the material composition and the temperature and time of the isothermal or two-step treatment applied to the biosilicate.

Still, the preparation of bioactive silicates of different crystalline phases makes possible the particle dissolution rate control, this being interesting under the point of view of the treatment of oral ailments.

**Compositions**

Under another aspect the present invention deals with the silica-based compositions used in the present process.

In view of the fact that the compositions that originate the crystalline particulate biosilicates of the invention are designed to be used in treatments of oral ailments, it is evident that all the components should be used in a relatively pure form, that is, free from highly toxic elements such as arsenic, lead, mercury and radioactive elements, for example, the components should be manipulated, melted, and thermally treated in aseptic environments.

Broadly, the particulate biosilicate compositions of the invention are according to Table 1 below, in weight % .

**TABLE 1**

| Component | Weight % |
|---|---|
| $SiO_2$ : | 40-60% |
| $Na_2O$: | 0-30% |
| CaO: | 15-30% |
| $K_2O$ : | 0-30% |
| $CaF_2$ : | 0-15% |
| NaF: | 0-15% |
| $Li_2O$: | 0-10% |
| SnO: | 0-10% |
| SrO: | 0-10% |
| $P_2O_5$ : | 0-8% |
| $Fe_2O_3$: | 0-6% |
| MgO: | 0-3% |
| $B_2O_3$: | 0 - 3% |
| $Al_2O_3$: | 0 - 3% |
| ZnO: | 0 - 3% |

The composition content, crystalline phases and the particle size determine the biosilicate biological activity and reaction kinetics with the dental tissue, the biosilicates being bioactive or resorbable.

Thus, for the crystalline phases of relatively slower reaction rates, reaction stages I to V described above in the present specification and related to the bioactive glass reactions with the body fluids are totally obeyed for the bioactive crystalline silicates of the invention, provided they are prepared from compositions formulated according to the desired end product and the processing of same being adequately adjusted.

On the other hand, for crystalline phases of quicker reaction rates with the tissue, that constitute the resorbable crystalline silicates, those are progressively degraded and replaced by the surrounding tissue, this resulting in the complete regeneration of the tissue, this being true, in the same way, provided the products are prepared from compositions formulated according to the desired end product and the processing of the compositions being adequately adjusted.

A typical composition according to the invention is illustrated in Table 2 below, with figures in weight % .

**TABLE 2**

| | |
|---|---|
| $SiO_2$ | 47.8 % |
| $Na_2O$ | 23.4% |
| CaO | 19.8% |
| $CaF_2$ | 5.0 % |
| $P_2O_5$ | 4.0 % |

A further typical composition of the invention in weight % is illustrated in Table 3 below, where the sodium content has been completely replaced by potassium.

**TABLE 3**

| | |
|---|---|
| $SiO_2$ | 48.5 % |
| $K_2O$ | 23.75 % |
| CaO | 23.75% |
| $P_2O_5$ | 4.0 % |

Still a typical composition of the invention, equally set forth in weight % is illustrated in Table 4 below.

**TABLE 4**

| | |
|---|---|
| $SiO_2$ | 48.5 % |
| $Na_2O$ | 23.75% |
| CaO | 23.75% |
| $P_2O_5$ | 4.0 % |

And Table 5 below illustrates still a typical composition of the invention, also in weight %.

**TABLE 5**

| | |
|---|---|
| $SiO_2$ | 44.9% |
| $Na_2O$ | 22.0% |
| CaO | 22.0% |
| $Li_2O$ | 7.4% |

(continued)

| | |
|---|---|
| $P_2O_5$ | 3.7% |

In view of the fact the $K^+$ ion release rate in contact with the body fluids is higher than that of the $Na^+$ ion, the potassium-containing compositions show a higher biological activity than those of the same sodium content.

**Particulate bioactive or resorbable silicates**

Under a further aspect, the invention relates to the particulate bioactive or resorbable crystalline silicates obtained by the present process.

According to the concept of the invention, bioactive silicates are those products that have the ability to bind to the dental tissue, forming HCA.

Analogously, such biosilicates are resorbable if not only they bind to the tooth but also allow that the tissue adjacent to the tooth progresses over the biosilicate particle and after a period the biosilicate is replaced by tissue, that is, the biosilicate material vanishes, being absorbed by the organism and replaced by tissue.

The basic feature of the bioactive silicates produced according to the invention is the ability to form hydroxycarbonate apatite on the surface of same after one day exposition in an artificial blood plasma known as simulated body fluid (SBF K-9) at 36.7°C, as cited in an article by Kokubo T. et al. "Solutions able to reproduce in vivo surface-structure changes in bioactive glass-ceramic A/W", *in* Journal of Biomedical Materials Research, v. 24, n° 6, p. 721-734, June 1990. The SBF K-9 fluid consists of a solution containing all the ions present in the human blood plasma in concentrations very close to those present in plasma.

For bioactivity tests with biosilicates the molten liquid above 1200°C should be poured into molds having a cylindrical shape. The so-obtained glass cylinders are annealed in an oven at temperatures varying between 400°C and 630°C according to the specific composition. Then, the cylinders are cut into disks with the aid of a diamond-recovered blade. The disks are directed to an oven for the thermal treatment in order to promote the practically complete crystallization of same. The biosilicate crystallization is obtained according to the processes described herein before.

The crystallized disks are placed each in a SBF K-9 fluid-containing capped polyethylene flask. The volume of the SBF K-9 solution (VS) in the flask in contact with the disk surface area (SA) should obey to the ratio

$$SA/VS = 0.1 cm^{-1}.$$

The disk should be suspended with the aid of a fine nylon thread attached to the flask cap so that none of the surfaces touches the flask. The flask should be completely sealed to avoid liquid evaporation and keep constant the solution concentration. After different exposition times, the disks are withdrawn from the flasks and analyzed in an infrared instrument equipped with Fourier transform (FTIR) for the evaluation of the surface changes.

Infrared spectroscopy with Fourier transform is used in the context of the present invention since it is a technique for surface analyses that is versatile for analyzing variations in the glass surface chemistry and for following surface changes in bioactive materials. The FTIR technique provides information on the chemical composition, the vibrational Si-O-Si and P-O modes of the surface, and can equally detect phase variations occurring in a layer of a certain surface. For example, the crystallization of a HCA layer on the surface of a bioactive material is easily detected by FTIR.

The molecular vibrations in the area of interest for the bioactive materials are listed in Table 6 below.

**TABLE 6**

| Wave number (cm$^{-1}$) | Vibrational Mode |
|---|---|
| 1350-1080 | P=O stretching |
| 1250-1100- | P=O associated |
| 940-860 | Si-O-Si stretching |
| 890-800 | C-O stretching |
| 1175-710 | Si-O-Si tetrahedral |
| 610-600 | P-O crystalline bending |
| 560-550 | P-O amorphous bending |
| 530-515 | P-O crystalline bending |
| 540-415 | Si-O-Si bending |

Figure 1 illustrates the FTIR spectra after the "in vitro" test in SBF K-9 of one of the crystalline biosilicates of the invention, having the initial composition illustrated in Table 7 below, with the composition in weight % , before the thermal treatment.

**TABLE 7**

| | |
|---|---|
| SiO$_2$ | 48.5% |
| Na$_2$O | 23.75 % |
| CaO | 23.75 % |
| P$_2$O$_5$ | 4.0 % |

The crystallized compositions that presented the formation of hdyroxycarbonate apatite in their surface in up to 72 hours immersion in SBF K-9 fluid were then wet milled using isopropyl alcohol or acetone in a high energy planetary mill using a jar and agate balls for periods between 100 and 200 minutes, depending on the specific composition, for obtaining a powder having particle size distribution between 0.1 and 30 microns. The powder mass / alcohol volume ratio used was 100g / 70 ml. In view of the ability of the biosilicates to react in aqueous media, the milling was carried out in analytical grade isopropyl alcohol or acetone.

The so-obtained crystalline powders were then placed in a polyethylene flask containing different gels used as vehicles in tooth pastes for the study of the powder's surface transformations after long residence times in these media. The objective of this test is to check the powder stability in different aqueous media, in concentrations and time that simulate the storing conditions of oral hygiene products. The aqueous medium volume / powder mass ratio varied between 10 ml/1 g to 100 ml / 0.5 g.

After powder exposition periods to the gels between one day and 90 days the suspensions are filtered under reduced pressure using filtering membranes of pore width 0.22 micron. The powder is oven-dried at 60°C. Powder- KBr disks are prepared in the 1/100 ratio and the disks are analyzed with the aid of infrared spectroscopy with Fourier transform.

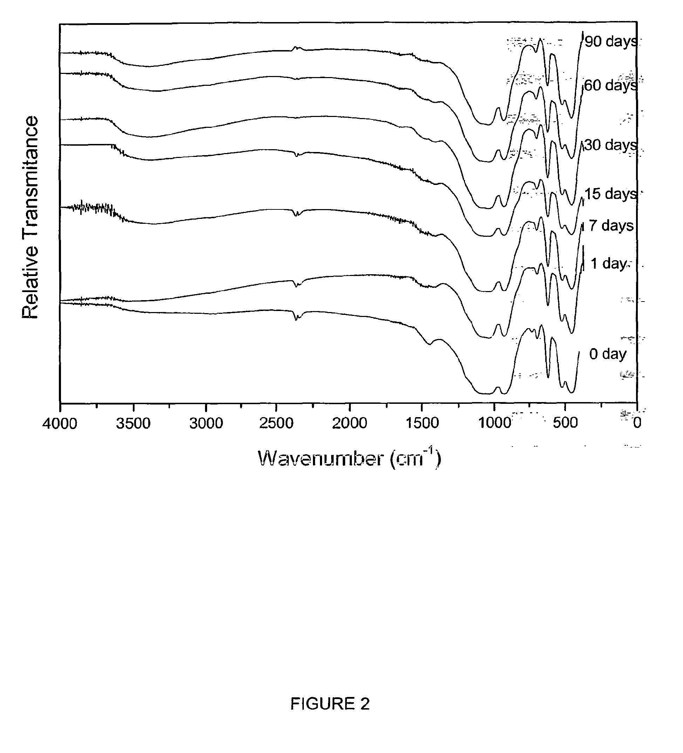

Figure 2 illustrates the infrared spectra for a particulate biosilicate exposed in one of the gels in the concentration of 30 ml / 1 g during 1, 7, 15, 30, 60 and 90 days.

From Figure 2 it can be noticed that the surface transformations occurred in the powder are very smooth and therefore even after 90 days exposition times the chemical feature of this powder surface is kept practically intact and therefore its ability to binding to bone tissues. The mild alteration occurred in the powder surface is not sufficient to modify the kinetics of the reactions occurring between the powder and the bone tissues and, as observed in the infrared curves, those are completely stabilized after 15 days of the powder rest in the gel. It should be noticed that in Figure 2 the peaks at 2366 and 2346 cm$^{-1}$ do not bear any relationship with the sample features, corresponding to the CO$_2$ variation in the environment of the experiment.

A further test allows to evaluate the ability of the crystallized powder to bind itself to the tooth surface.

In this test, a tooth is cleaned with distilled water and fixed to an apparatus that simulates teeth brushing. A biosilicate suspension is prepared having particle size distribution varying between 0.1 and 20 microns in distilled water at the ratio 1 g / 100 ml. The brushing frequency used is 365 rpm during 20 seconds. The course traversed by the brush corresponds to 3.8 cm. The weight of the shoe with coupled brush is 200 g. After the brushing simulation, the tooth is placed in distilled water for 20 hours. The tooth surface is then dried with a hot air flaw and analyzed with the aid of scanning electronic microscopy.

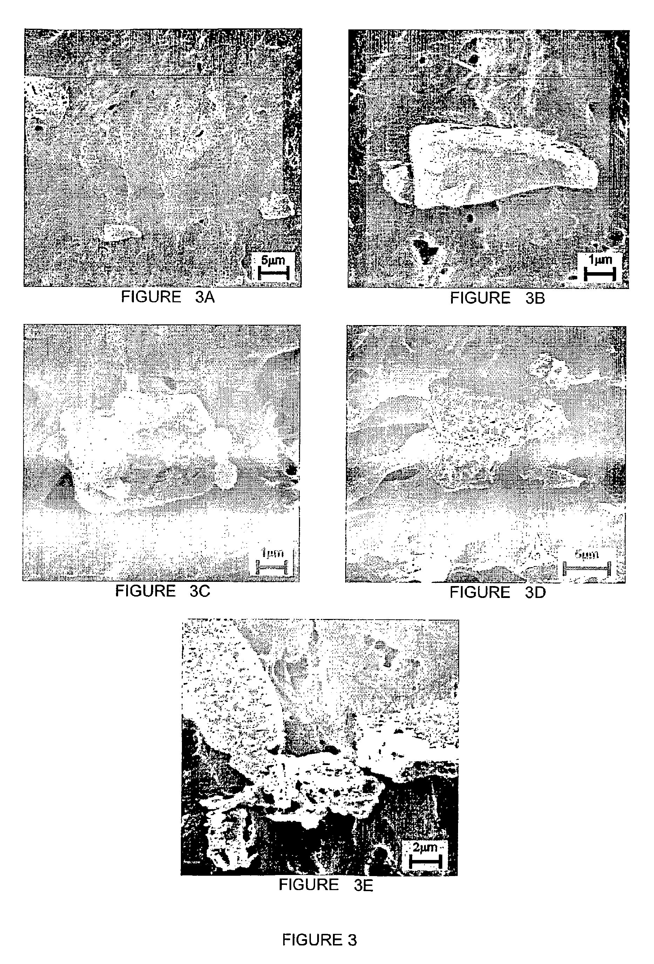

Figure 3 illustrates for several electronic microscope magnifications the biosilicate particles on the tooth surface, more specifically, of the cervical tooth area.

The micrographs shown in Figure 3 allow to observe that the biosilicate particles start a sealing process with the tooth surface, but the contact between such particles and the tooth is not well defined, showing a smooth binding interface between the particles and the tooth surface. Most of the particles show deeply eroded surfaces (Figures 3A, 3C, 3D and 3E), with a trend to be gradually replaced by the tissue in contact, their surface being occupied by collagen fibrils. This is because the cervical area possesses a certain amount of these fibrils, that are easily bound to bioactive or resorbable materials. The area of most of the particles not in contact with the tooth surface tends to be completely dissolved. In this way, $Si^{4+}$, $Ca^{2+}$, $P^{5+}$, $F^-$ and $K^+$ ions are released in solution, aiding in the binding processes of the particles to the tooth surface and in remineralization processes. Therefore, the biosilicate particles in this particle size range (20 to 0.1 micron) are efficient for the purposes of the invention.

A relevant further additional feature relative to a biosilicate for use in the treatment of oral ailments is the fact that it shows bactericidal properties.

Then, tests are run in order to check if the biosilicates of the invention satisfy this requirement. In this test, 1 g of the powdered biosilicate having a particle size distribution between 20 and 0.1 micron is placed in 30 ml of aqueous solution. The same amount of powdered bioactive glass of same particle size distribution is placed in 30 ml of the same liquid. Under these conditions the pH change of the liquid with exposition time is followed.

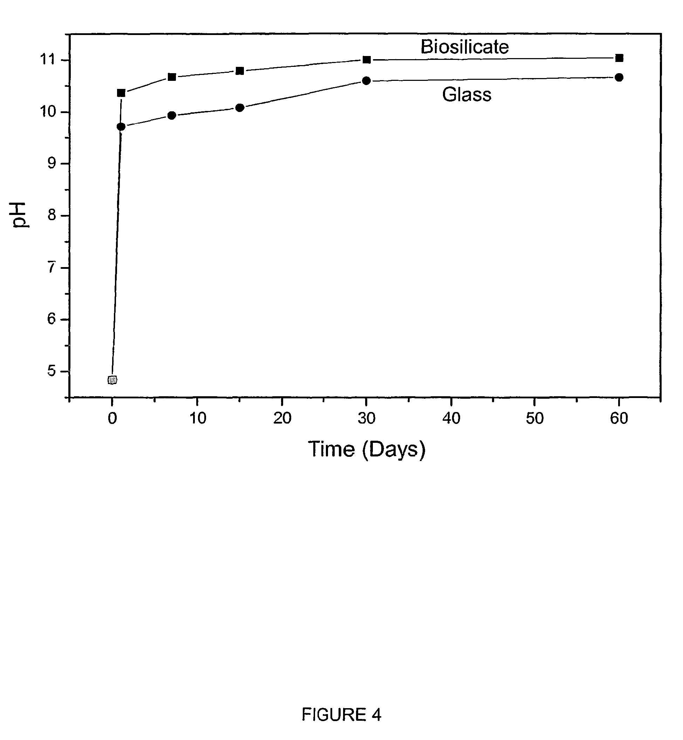

As seen in the graph of Figure 4, the inventive biosilicates show the same bactericidal properties as state-of-the-art bioactive glasses, since they undergo the same hydroxycarbonate apatite layer forming processes and release the same ions in solution than the bioactive glasses, providing a still higher pH rise with exposition time in solution than the non-crystallized glass of same composition and particle size distribution.

Thus is secured the efficacy of the biosilicates to combat pathogen bacteria.

**Use of the particulate bioactive or resorbable biosilicates in the treatment of oral ailments**

A still further aspect of the present invention is the use of the particulate bioactive or resorbable biosilicates in the treatment of oral ailments.

The crystalline particulate bioactive or resorbable biosilicates according to the invention have different reaction rates with the saliva and the dental tissue depending on the particle size and crystalline phase.

For the purposes of the invention the particle size of the bioactive silicates varies between 30 and 0.1 micron. A preferred range is between 20 and 0.1 micron.

Such particles can be incorporated into vehicles, such as tooth pastes, gels, mouth washings, oral antiseptics, saline solution, artificial saliva, etc. The amount of biosilicate to be incorporated in these vehicles depend on the vehicle and on the specific treatment and is between 0.5 and 10wt %. A preferred range for this amount is between 0.5 and 3wt% for incorporating into gels and oral antiseptics.

The biosilicate particles are distributed to the dental tissues during brushing or by practicing oral hygiene, but they can also be applied in clinics for dental treatment.

The particles between 4 and 0.1 micron have the ability to penetrate the dentine tubules or any microfissure and at first to establish a strong chemical bond between the dental tissue and be gradually replaced by the surrounding tissue, promoting the occlusion of these tubules and fissures, and consequently, eliminating the sensitivity in these areas.

Particles between 30 and 4 microns are designed to deposit on the teeth and dentine outer surface. In view of their ability of quickly reacting with the tooth tissue and forming hydroxycarbonate apatite, making thus a strong chemical bond between the particles and the tissue, such particles can work as calcium and phosphorus ion suppliers for the reaction of the smaller particles positioned inside the tubules, by dissolution that can be controlled using different crystalline phases that show different solubility rates.

Besides, these particles are sources of $Ca^{2+}$, $P^{5+}$ and $F^-$ ions for the saliva for prolonged periods, this favoring the tooth remineralization processes, fissure calcification and incipient cavities as well as dental tissue regeneration.

Added to this there are the effects of pH rise provided for by $Na^+$, $Ca^{2+}$ and $K^+$, since these ions provide a hostile environment for the survival of pathogen bacteria, showing a bactericidal effect as described in US patents 6,342,207 and US 6,190,643, avoiding cavity, gingivitis and bacterial plaque proliferation, and thus promoting the maintenance of oral health.

Besides these beneficial effects for the oral health, the constant release of $K^+$ in the saliva provides a concentration rise of these ions in the surroundings of the pulp mechano-receptors, through diffusion of these ions towards the interior of the dentine tubules. This results in an analgesic effect to the dentine sensibility, before all the dentine tubules have not been obstructed.

The quick and strong bond of the particles with the tooth avoids that the particles be entrained by the saliva movement or by the practice of oral hygiene.

Successive applications of the biosilicate to the tooth allow the formation of a layer made up of hydroxycarbonate apatite particles on the tooth and dentine or still the complete tooth surface regeneration.

Smaller biosilicate particles (diameter < 1 micron) can deposit on microfissures, and their high solubility in saliva causes a sharp increase of the local calcium and phosphorus concentration, this contributing to the re-calcification of these fissures.

The presence of crystalline phases with fluorine of different solubility rates are also very important for the recovery of lesions and the promotion of oral health, since fluorine, even at small concentrations in the saliva, provides for the precipitation of calcium-rich crystalline phases on the tooth surface.

According to the publication by Cruz, R. A. "Considerações clinicas e laboratoriais sobre a reatividade de compostos fluoretados aplicados topicamente no esmalte dental humano". In: Kriger, L. ABOPREV: Promoção de saúde bucal. 2. ed., São Paulo: Artes Médicas, 1999. p. 168-194, ( *Clinical and Laboratorial Considerations on the Reactivity of Fluoride Compounds Topically Applied to the Human Dental Enamel" in* Kriger, L ABOPREV : *"Promotion of the oral health. 2nd edition, São Paulo, Medical Arts,* 1999, *p. 168-194),* the application of a 0.05% NaF solution for 30 seconds is sufficient for forming a $CaF_2$ globular layer on the enamel surface. Longer exposition times to the solution cause a rise in the deposited fluoride amount.

Based on recent research data, the deposition of such $CaF_2$ layer on the enamel surface and on the incipient lesions of micropores should be responsible for the beneficial action of dental treatment with fluorine. Fluorine released by fluoride globules could stabilize the enamel surface, increase the resistance against acids or potentialize remineralization during the pH cycles in the oral environment.

A further application of the inventive biosilicates is in the treatment of xerostomy, where an alteration of the dental tissue occurs by loss of the tooth mineral phase in patients having diminished saliva caused by radiotherapy cancer treatment.

**Conclusion**

The inventive biosilicates have the ability of withstanding surface modifications when inserted in oral hygiene vehicles, showing sufficient chemical resistance to stand long residence times in aqueous solutions without losing their properties for reacting with dental tissues. On the other hand, for state-of-the-art bioactive glasses no data can be found related to the chemical resistance of same during long residence times in aqueous solution. Thus, the bioactive silicates of the invention have a long shelf life when conditioned in oral hygiene products.

Contrary to the state-of-the-art tooth pastes used for the desensitization of the dentine that contain potassium nitrate and show only analgesic effect on the sensitivity, the inventive biosilicates, in view of their ability to bind to dental tissues causing their regeneration and coverage, promote the occlusion of the dentine tubules in a long lasting manner, with the treatment being interrupted after a certain number of applications. Besides, for the potassium-containing compositions such ions are constantly released during the surface leaching, promoting the same analgesic effect on the mechano-receptors of the pulp nerves.

By forming an interfacial hydroxycarbonate apatite layer of same chemical composition of the teeth mineral phase, the inventive bioactive silicates establish a strong chemical bond with the dental tissues.

Advantageously, the described and claimed biosilicates have the ability to favor the teeth remineralization processes. When present in the oral environment, the biosilicate particle surface start a leaching process by releasing $Ca^{2+}$ e $P^{5+}$ ions that are absorbed by the tooth hydroxyapatite crystals, and $F^-$, that stabilize tooth hydroxyfluorapatite phases, these being chemically more resistant and also promote the formation of $CaF_2$ globules on the tooth surface, these globules also working as a fluorine reserve for hydroxyfluorapatite phase stabilization. After the quick biosilicate bond to the tooth the second beneficial triggered stage is the slow release of such ions in the oral environment providing for the maintenance of high levels of such ions for prolonged periods. This way is secured a favorable environment for healthier teeth.

Thus, the biosilicates are advantageous over tooth pastes that use slightly soluble calcium and phosphorus salts to promote the tooth remineralization, since this slow process requires the prolonged presence of these ions sources in the oral environment to efficiently promote remineralization.

Biosilicates containing particles of up to 0.1 micron can further promote fissure sealing and reduce porosity of a tooth in case of tooth whitening using peroxides. In peroxide-treatments, the breaking of the tooth organic molecules beyond the saturation level causes an increase in the tooth porosity, rendering it more fragile and brittle. Besides, the pores resulting from whitening are sites for deposition of new organic molecules that cause the tooth darkening.

Contrary to the limitations of state-of-the-art processes and products, the process for preparing biosilicates according to the teachings of the invention leads to particles of quick reactivity with the dental tissue for treating dentine sensitivity and microfissures, or particles of less soluble crystalline phases, that can stand for prolonged periods in the oral environment, with the purpose to promote tooth remineralization. This combined effect is highly beneficial for maintaining oral health while at the same time causing the elimination of pathogen bacteria.

Thermal treatments according to the invention modes generate biosilicates that are practically free from vitreous phase, this permitting their milling for obtaining particles devoid of sharp and cutting surfaces, these being surfaces that cause minimal harm to gum and mucosae during the practice of oral hygiene, these features allowing to incorporate the inventive biosilicates to gels and dental creams designed to tooth brushing.

The above description proves therefore that from different starting compositions and by varying the conditions of thermal treatment, the inventive process in its two modes allows to obtain a wide variety of different biosilicates, of diverse compositions and crystalline phases, that can be employed as such or combined, providing the therapeutic use of these biosilicates in a broad range of oral ailments.

**Claims**

1. A process for preparing particulate, bioactive, crystalline and resorbable biosilicates from vitreous plates or frits, wherein the process comprises the following steps:

   a) Submitting the vitreous plates or frits to a one-step thermal treatment with the simultaneous promotion of crystal nucleation and growth, so as to obtain crystalline silicates;
   b) Milling the so-obtained crystalline silicates to obtain a particle size distribution between 0.1 and 30 $\mu$m;
   c) Recovering the particulate bioactive, crystalline and resorbable biosilicate product having not more than 0.5% residual vitreous phase, whereby said crystalline, bioactive, particulate biosilicate product is free from conchoidal fracture.

2. A process according to claim 1, wherein alternatively the milling step precedes the thermal treatment.

3. A process according to claim 1, wherein alternatively the thermal treatment is performed in two steps.

4. A process according to claim 3, wherein alternatively the milling step precedes the thermal step.

5. A process according to claims 1, 2, 3 and 4, wherein the crystalline phases formed during the isothermal or two-step thermal treatment comprise $1Na_2O.2CaO.3SiO_2$, $2Na_2O.1CaO.3SiO_2$, $Na_2OCa(PO_4)F$, and $Ca_5Si_6O_{16}(OH)_2.8H_2O$.

6. A process according to claims 1 and 5, wherein said crystalline phases have different dissolution rates and biological activity, the crystalline phases with higher dissolution rate reacting faster with the tooth tissue and causing the tooth structure to regenerate while the crystalline phases with lower dissolution rate binding to the tooth tissue through HA formation.

7. A process according to claim 1, wherein the bioactive silicate particle size is between 0.1 and 20 $\mu$m.

8. A process according to claim 7, wherein the bioactive silicate particle size is between 0.1 and 4 $\mu$m.

9. A process according to claims 1 and 8, wherein the bioactive silicate particle size is between 30 and 4 $\mu$m.

10. A process according to claims 7 and 8, wherein the bioactive silicate particle size is <1 $\mu$m.

11. Compositions for preparing the biosilicates according to the process of claims 1, 2, 3 and 4, wherein such compositions comprise, in weight %, between 40 and 60% $SiO_2$, between 0 and 30% $K_2O$, between 0 and 30 % $Na_2O$, between 0 and 15% NaF, between 15 and 30% CaO, between 0 and 15% $CaF_2$, between 0 and 10% $Li_2O$, between 0 and 10% SnO, between 0 and 10% SrO, between 0 and 8% $P_2O_5$, between 0 and 6% $Fe_2O_3$, between 0 and 3% MgO, between 0 and 3% $B_2O_3$, between 0 and 3% $Al_2O_3$ and between 0 and 3% ZnO.

12. Compositions according to claim 11, wherein said compositions comprise, in weight %:

    | | |
    |---|---|
    | $SiO_2$ | 47.8 % |
    | $Na_2O$ | 23.4% |
    | CaO | 19.8% |
    | $CaF_2$ | 5.0 % |
    | $P_2O_5$ | 4.0 % |

13. Compositions according to claim 11, wherein said compositions comprise, in weight %:

    | | |
    |---|---|
    | $SiO_2$ | 48.5 % |
    | $K_2O$ | 23.75% |
    | CaO | 23.75% |
    | $P_2O_5$ | 4.0 % |

14. Compositions according to claim 11, wherein said compositions comprise, in weight %:

    | | |
    |---|---|
    | $SiO_2$ | 44.9% |
    | $Na_2O$ | 22.0% |
    | CaO | 22.0% |
    | $Li_2O$ | 7.4% |
    | $P_2O_5$ | 3.7% |

15. Particulate, bioactive, crystalline and resorbable biosilicates, wherein said biosilicates are prepared according to the process of claims 1, 2, 3 and 4.

16. Biosilicates according to claim 15, wherein said biosilicates are free from conchoidal fracture.

17. Biosilicates according to claim 15, wherein said biosilicates are included in a dental gel in an amount between 0.5 and 10% by weight, more preferably between 0.5 and 3.0 % by weight.

**Patentansprüche**

1. Verfahren zur Herstellung partikulärer, bioaktiver, kristalliner und resorbierbarer Biosilikate aus glasartigen Platten oder Fritten, wobei das Verfahren die folgenden Schritte umfasst:

    a) Unterziehen der glasartigen Platten oder Fritten einer einstufigen Thermalbehandlung mit der gleichzeitigen Förderung von Kristallkeimbildung und - wachstum, um somit kristalline Silikate zu erhalten;
    b) Zermahlen der so erhaltenen kristallinen Silikate, um eine Partikelgrößenverteilung zwischen 0.1 und 30 $\mu$m zu erhalten;
    c) Gewinnen des partikuläreren, bioaktiven, kristallinen und resorbierbaren Biosilikat-Produkts,

    wobei das partikulärere, bioaktive, kristalline und resorbierbare Biosilikat-Produkt frei von muscheligem Bruch ist.

2. Verfahren nach Anspruch 1, wobei der Zermahlungsschritt alternativ der Thermalbehandlung vorausgeht.

3. Verfahren nach Anspruch 1, wobei die Thermalbehandlung alternativ zweistufig ist.

4. Verfahren nach Anspruch 3, wobei der Zermahlungsschritt alternativ der Thermalbehandlung vorausgeht.

5. Verfahren nach den Ansprüchen 1, 2, 3 und 4, wobei die kristallinen Phasen, die während der isothermen oder zweistufigen thermischen Behandlung gebildet werden, $1Na_2O.2CaO.3SiO_2$, $2Na_2O.1CaO.3SiO_2$, $Na_2OCa(PO_4)F$ und $Ca_5Si_6O_{16}(OH)_2.8H_2O$ umfassen.

6. Verfahren nach Ansprüchen 1 und 5, wobei die kristallinen Phasen unterschiedliche Auflösungsgeschwindigkeiten und biologische Aktivitäten aufweisen; wobei die kristallinen Phasen mit höherer Auflösungsgeschwindigkeit schneller mit dem Zahnfleisch reagieren und eine Regeneration der Zahnstruktur bewirken, wohingegen die kristallinen Phasen mit niedrigerer Auflösungsgeschwindigkeit sich durch HA-Bildung an das Zahnfleisch binden.

7. Verfahren nach Anspruch 1, wobei die Partikelgröße der bioaktiven Silikate zwischen 0,1 und 20 $\mu$m liegt.

8. Verfahren nach Anspruch 7, wobei die Partikelgröße der bioaktiven Silikate zwischen 0,1 und 4 $\mu$m liegt.

9. Verfahren nach Ansprüchen 1 und 8, wobei die Partikelgröße der bioaktiven Silikate zwischen 30 und 4 $\mu$m liegt.

10. Verfahren nach Ansprüchen 7 und 8, wobei die Partikelgröße der bioaktiven Silikate < 1 $\mu$m ist.

11. Zusammensetzungen zur Darstellung der Biosilikate nach dem Verfahren der Ansprüche 1, 2, 3 und 4, wobei solche Zusammensetzungen in Gewichts-% zwischen 40 und 60 % $SiO_2$, zwischen 0 und 30 % $K_2O$, zwischen 0 und 30 % $Na_2O$ zwischen 0 und 15 % NaF, zwischen 15 und 30 % CaO, zwischen 0 und 15 % $CaF_2$, zwischen 0 und 10 % $Li_2O$, zwischen 0 und 10 % SnO, zwischen 0 und 10 % SrO, zwischen 0 und 8 % $P_2O_5$, zwischen 0 und 6 % $Fe_2O_3$, zwischen und 3% MgO, zwischen 0 und 3 % $B_2O_3$, zwischen 0 und 3 % $Al_2O_3$ und zwischen 0 und 3 % ZnO umfassen.

12. Zusammensetzung nach Anspruch 11, wobei diese Zusammensetzungen in Gewichts-% umfassen:

    | | |
    |---|---|
    | $SiO_2$ | 47.8 % |
    | $Na_2O$ | 23.4 % |
    | CaO | 19,8 % |
    | $CaF_2$ | 5,0 % |
    | $P_2O_5$ | 4,0 % |

13. Zusammensetzung nach Anspruch 11, wobei diese Zusammensetzungen in Gewichts-% umfassen:

    | | |
    |---|---|
    | $SiO_2$ | 48,5% |
    | $K_2O$ | 23,75% |
    | CaO | 23,75% |
    | $P_2O_5$ | 4,0% |

14. Zusammensetzung nach Anspruch 11, wobei diese Zusammensetzungen in Gewichts-% umfassen:

    | | |
    |---|---|
    | $SiO_2$ | 44,9% |
    | $Na_2O$ | 22,0 % |
    | CaO | 22,0 % |
    | $Li_2O$ | 7,4% |
    | $P_2O_5$ | 3.7% |

15. Partikuläre, bioaktive, kristalline und resorbierbare Biosilikate, wobei die Biosilikate nach dem Verfahren gemäß den Ansprüchen 1, 2, 3 und 4 hergestellt sind.

16. Biosilikate nach Anspruch 15, wobei die Biosilikate frei von muscheligem Bruch sind.

**17.** Biosilikate nach Anspruch 15, wobei die Biosilikate in einer Menge zwischen 0,5 und 10 Gewichts-%, vorzugsweise zwischen 0,5 und 3,0 Gewichts-%, von einem Dentalgel beinhaltet sind.

**Revendications**

**1.** Procédé de préparation de biosilicates particulaires, bioactifs, cristallins et résorbables à partir de plaques ou de frittes vitreuses, dans lequel le procédé comprend les étapes suivantes consistant à :

a) soumettre les plaques ou frittes vitreuses à un traitement thermique à une étape avec la promotion simultanée de la germination et de la croissance cristalline, de façon à obtenir des silicates cristallins ;
b) broyer les silicates cristallins ainsi obtenus pour obtenir une distribution de taille de particule entre 0,1 et 30 $\mu$m ;
c) récupérer le produit de biosilicate particulaire, bioactif, cristallin et résorbable n'ayant pas plus de 0,5 % de phase vitreuse résiduelle, moyennant quoi ledit produit de biosilicate cristallin, bioactif et particulaire est dépourvu de cassure conchoïdale.

**2.** Procédé selon la revendication 1, dans lequel en variante l'étape de broyage précède le traitement thermique.

**3.** Procédé selon la revendication 1, dans lequel en variante le traitement thermique est réalisé en deux étapes.

**4.** Procédé selon la revendication 3, dans lequel en variante l'étape de broyage précède l'étape thermique.

**5.** Procédé selon les revendications 1, 2, 3 et 4, dans lequel les phases cristallines formées pendant le traitement isothermique ou thermique à deux étapes comprennent $1Na_2O.2CaO.3SiO_2$, $2Na_2O.1CaO.3SiO_2$, $Na_2OCa(PO_4)F$ et $Ca_5Si_6O_{16}(OH)_2.8H_2O$.

**6.** Procédé selon les revendications 1 et 5, dans lequel lesdites phases cristallines ont des vitesses de dissolution et une activité biologique différentes, les phases cristallines ayant une vitesse de dissolution plus élevée réagissant plus rapidement avec le tissu dentaire et amenant la structure dentaire à se régénérer alors que les phases cristallines ayant une vitesse de dissolution plus faible se liant au tissu dentaire à travers une formation de HA.

**7.** Procédé selon la revendication 1, dans lequel la taille de particule des silicates bioactifs est entre 0,1 et 20 $\mu$m.

**8.** Procédé selon la revendication 7, dans lequel la taille de particule des silicates bioactifs est entre 0,1 et 4 $\mu$m.

**9.** Procédé selon les revendications 1 et 8, dans lequel la taille de particule des silicates bioactifs est entre 30 et 4 $\mu$m.

**10.** Procédé selon les revendications 7 et 8, dans lequel la taille de particule des silicates bioactifs est inférieure à 1 $\mu$m.

**11.** Compositions pour préparer les biosilicates selon le procédé des revendications 1, 2, 3 et 4, dans lesquelles ces compositions comprennent, en % en poids, entre 40 et 60 % de $SiO_2$, entre 0 et 30 % de $K_2O$, entre 0 et 30 % de $Na_2O$, entre 0 et 15 % de NaF, entre 15 et 30 % de CaO, entre 0 et 15 % de $CaF_2$, entre 0 et 10 % de $Li_2O$, entre 0 et 10 % de SnO, entre 0 et 10 % de SrO, entre 0 et 8 % de $P_2O_5$, entre 0 et 6 % de $Fe_2O_3$, entre 0 et 3 % de MgO, entre 0 et 3 % de $B_2O_3$, entre 0 et 3 % de $Al_2O_3$ et entre 0 et 3 % de ZnO.

**12.** Compositions selon la revendication 11, dans lesquelles lesdites compositions comprennent, en % en poids :

| | |
|---|---|
| $SiO_2$ | 47,8% |
| $Na_2O$ | 23,4% |
| CaO | 19,8% |
| $CaF_2$ | 5,0 % |
| $P_2O_5$ | 4,0 % |

**13.** Compositions selon la revendication 11, dans lesquelles lesdites compositions comprennent, en % en poids :

| | |
|---|---|
| $SiO_2$ | 48,5 % |
| $K_2O$ | 23,75 % |
| CaO | 23,75 % |
| $P_2O_5$ | 4,0 % |

**14.** Compositions selon la revendication 11, dans lesquelles lesdites compositions comprennent, en % en poids :

| | |
|---|---|
| $SiO_2$ | 44,9 % |
| $Na_2O$ | 22,0 % |
| CaO | 22,0 % |
| $Li_2O$ | 7,4 % |
| $P_2O_5$ | 3, 7 % |

**15.** Biosilicates particulaires, bioactifs, cristallins et résorbables, dans lesquels lesdits biosilicates sont préparés selon le procédé des revendications 1, 2, 3 et 4.

**16.** Biosilicates selon la revendication 15, dans lesquels lesdits biosilicates sont dépourvus de cassure conchoïdale.

**17.** Biosilicates selon la revendication 15, dans lesquels lesdits biosilicates sont compris dans un gel dentaire en une quantité comprise entre 0,5 et 10 % en poids, de manière davantage préférée entre 0,5 et 3,0 % en poids.

FIGURE 1

FIGURE 2

FIGURE 3A

FIGURE 3B

FIGURE 3C

FIGURE 3D

FIGURE 3E

FIGURE 3

FIGURE 4

FIGURE 5